(19) Europäisches Patentamt
European Patent Office
Office européen des brevets

(11) Numéro de publication: **0 138 931 B1**

(12) # FASCICULE DE BREVET EUROPEEN

(45) Date de publication du fascicule du brevet: **01.06.88**

(51) Int. Cl.⁴: **A 61 K 7/48**

(21) Numéro de dépôt: **84901362.8**

(22) Date de dépôt: **23.03.84**

(86) Numéro de dépôt international: **PCT/FR 84/00079**

(87) Numéro de publication internationale: **WO 84/03836 (11.10.84** Gazette **84/24)**

(54) **COMPOSITION DERMATOLOGIQUE ET SON PROCEDE DE PREPARATION.**

(30) Priorité: **30.03.83 FR 8305282**

(43) Date de publication de la demande: **02.05.85 Bulletin 85/18**

(45) Mention de la délivrance du brevet: **01.06.88 Bulletin 88/22**

(84) Etats contractants désignés: **AT BE CH DE GB LI LU NL SE**

(56) Documents cités:
**BE - A - 855 118**
**FR - A - 2 191 878**
**FR - A - 2 249 651**
**FR - A - 2 275 193**

**THE MERCK INDEX, 10th edition, 1983, No. 1226, Rahway, N.J. (US), see page 174; Ref. 1226 Quercitrin Chemical Abstracts, Vol. 82, No. 13, 31 March 1975, Columbus, Ohio (US). A.J. Shrikhande et al.: "Effect of flavonols on ascorbic acid and anthocyanin stability in model systems", see page 389, abstract 84587a, J. Food Sci. 1974, 39(5), 904-6 (Eng.)**

**Le dossier contient des informations techniques présentées postérieurement au dépôt de la demande et ne figurant pas dans le présent fascicule.**

(73) Titulaire: **DURAFFOURD, Alain, 11 bis, avenue Victor Hugo, F-75116 Paris (FR)**
Titulaire: **DURAFFOURD, Jean-Max, 11 bis, avenue Victor Hugo, F-75116 Paris (FR)**

(72) Inventeur: **DURAFFOURD, Alain, 11 bis, avenue Victor Hugo, F-75116 Paris (FR)**
Inventeur: **DURAFFOURD, Jean-Max, 11 bis, avenue Victor Hugo, F-75116 Paris (FR)**

(74) Mandataire: **Bouju, André, Cabinet Bouju 38 avenue de la Grande Armée, F-75017 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne une composition pour régénérer le collagène du tissu conjonctif de la peau.

Le collagène est le principal constituant (70% du tissu conjonctif) qui constitue le tissu de soutien des organes et en particulier de la peau.

Le collagène est composé de longues fibres élastiques polypéptidiques, reliées entre elles par des ponts, qui assurent la cohésion et la stabilité du tissu conjonctif. Cette texture lui permet de jouer son role de tissu élastique dans toutes les directions et de retenir l'eau pour participer activement à la régulation hydrique au niveau de la peau. Le vieillissement du collagène se traduit par une rupture des liaisons qui relient les fibres. L'âge, les intempéries, la pollution accélèrent ces ruptures et ralentissent leur renouvellement. De ce fait, la peau perd son élasticité et se déshydrate. Les relâchements de la peau qui en résultent provoquent la formation de rides et de plis inesthétiques.

Ces inconvénients ne sont pas seulement d'ordre esthétique. La peau n'est pas qu'une enveloppe; elle est un organe jouissant de propriétés multiples, indispensables au bon fonctionnement de tout l'organisme. A cet effet la peau est dotée d'un système vasculaire très développé qui est à la base des échanges indispensables à l'accomplissement des différentes fonctions de la peau. Les parois des capillaires de ce système vasculaire sont essentiellement constituées de tissu conjonctif. Son altération diminue l'élasticité des parois, modifiant ainsi les rapports de pression entre la partie artérielle et la partie veineuse, qui sont à la base du mécanisme qui assure les apports artériels et les retours veineux, donc la nutrition de la peau et les métabolismes spécifiques de son activité.

Sans préjudice des troubles que peuvent entraîner le ralentissement des différentes fonctions de la peau, la malnutrition de la peau se traduit par un vieillissement de cette dernière, qui est la conséquence de renouvellement insuffisant des cellules âgées.

Il est donc d'un intérêt doublement important de corriger ces déficiences fonctionnelles de la peau, puisqu'ainsi on agit sur la santé physique en redonnant à la peau son activité physiologique et sur la santé morale par l'aspect esthétique de cette correction.

On sait, selon le «Chemical Abstracts, volume 82, No 13, du 31 Mars 1975, abrégé 84587a, que les plantes renferment des substances flavonoïdes qui peuvent être extraites au moyen d'une solution aqueuse éventuellement additionnée d'alcool. Selon ce document, ces flavonoïdes présentent un effet thérapeutique qui est augmenté en présence de vitamine C.

Toutefois, ce document ne révèle pas que ces flavonoïdes ont une action positive sur les fibres de collagène.

On connaît, par ailleurs, selon le FR-A-2275193, un extrait aqueux de conifère utilisable en cosmétique.

Il est probable que cet extrait renferme des flavonols et de la vitamine C. Toutefois, ce document ne revèle pas la nature précise de tels flavonols, ni l'action de la composition sur les fibres du collagène.

De même, le FR-A-2249651 décrit une crème pour la peau contenant un extrait naturel qui renferme des substances flavonoïdes. La nature précise de ces substances n'est pas non plus précisée dans ce document.

Le but de la présente invention est de fournir une composition permettant de régénérer efficacement le collagène du tissu conjonctif de la peau.

Suivant l'invention, la composition pour régénérer le collagène du tissu conjonctif de la peau, renfermant un extrait aqueux d'écorces de conifères contenant des flavonols et de la vitamine C, est caractérisée en ce que l'extrait aqueux est tamponné à un pH voisin de 7 et les flavonols sont sous forme de dimères.

Les flavonols extraits des écorces de conifères tels que le pin et le cyprès présentent la formule suivante:

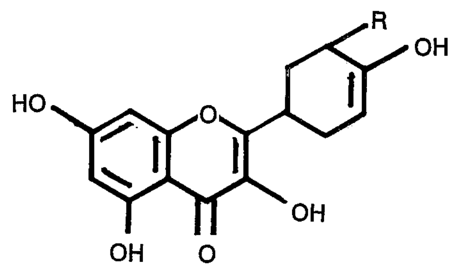

dans laquelle R désigne un radical alkyle inférieur.

Le demandeur a constaté de façon surprenante que ces flavonols, lorsqu'ils sont sous forme de dimères, avaient une action positive sur la régénération du collagène du tissu conjonctif.

Il a été constaté, d'autre part, que la présence la vitamine C dans l'extrait aqueux avait, non seulement pour effet d'apporter à l'organisme de la vitamine C, mais également de potentialiser les effets des flavonols.

Les flavonols extraits d'écorces de conifères ont tendance à se polymériser facilement en perdant leur activité sur la régénération du collagène.

La présence du tampon à pH voisin de 7 dans la composition évite cette polymérisation et permet de maintenir les flavonols en solution surtout sous la forme de dimères qui sont les plus actifs.

Les expériences effectuées sur des animaux ont montré que la composition conforme à l'invention diffusait parfaitement dans la peau et atteignait facilement non seulement le tissu conjonctif de la peau mais aussi celui des vaisseaux. L'application locale de la composition conforme à l'invention est donc parfaitement adaptée au traitement des déficiences du tissu conjonctif, dont ils constituent une correction spécifique et totale.

Suivant un autre aspect de l'invention, le procédé pour préparer la composition conforme à l'invention comprend les étapes suivantes:

a) on fait infuser à ébullition, puis macérer des écorces de conifères dans de l'eau additionnée d'alcool,

b) on filtre la suspension obtenue et on additionne un anti-oxydant naturel et un tampon pour maintenir le pH à une valeur voisine de 7,

c) on fait macérer des aiguilles de conifères dans de l'eau additionnée d'un tampon pour maintenir le pH à une valeur voisine de 7 puis on filtre,

d) on mélange le filtrat obtenu avec celui obtenu à la fin de l'étape b).

Grâce à ce mode opératoire, on extrait non seulement des quantités appréciables de flavonols, mais également on évite leur polymérisation.

De plus on obtient lors de l'étape c) une solution riche en vitamine C qui permet d'ajouter cette vitamine à la solution renfermant les flavonols qui est obtenue lors de l'étape b).

En tant que tampon on utilise de préférence du borate.

En tant qu'anti-oxydant on utilise de préférence de l'éthyle gallate qui présente l'avantage d'être naturel et d'être compatible avec les autres ingrédients de la composition et d'être acceptable par la peau et le tissu conjonctif.

On donne ci-après à titre d'exemple non limitatif, le mode opératoire de la préparation d'une composition conforme à l'invention.

Une infusion d'écorces de pin maritime est préparée à raison de 100 grammes d'écorces concassées pour 1 litre d'eau, additionnée de 50 ml d'alcool éthylique à 95°.

Cette infusion est maintenue à ébullition pendant 20 minutes. Cette infusion est suivie ensuite d'une macération de 2 heures dans la même eau alcoolisée.

La suspension obtenue et ensuite filtrée et additionnée d'un anti-oxydant naturel tel que de l'éthyle gallate, et d'un tampon borate à pH 7. Cette précaution est indispensable pour éviter une polymérisation des flavonols par acidification ou leur oxydation qui modifierait leurs propriétés.

Par ailleurs 100 grammes d'aiguilles sèches de pin maritime sont mises à macérer dans un litre d'eau, tamponnée à pH 7, pendant 2 heures. La suspension obtenue est filtrée. Le filtrat ainsi récupéré est alors mélangé au filtrat obtenu précédemment. La macération d'aiguilles de pins apporte non seulement un complément de principes actifs (falvonols) mais aussi de la vitamine C qui catalyse l'action des flavonols. Les flavonols contenus dans la solution obtenue est dosée à l'aide d'un réactif phosphotungstique étalonné. Le titre final est ajusté par dilution pour obtenir une activité constante.

En vue de son usage, la composition obtenue cidessus est diluée de façon à renfermer entre 1 à 5 grammes par litre de flavonols et 10 à 50 mg par litre de vitamine C.

De préférence, la composition renferme 2,5 grammes par litre de flavonols et 25 mg par litre de vitamine C.

La composition conforme à l'invention peut être appliquée sur la peau sous forme de lotions, de crèmes, de pommades, etc.

On donne ci-après quelques exemples de formulations cosmétiques de la composition conforme à l'invention.

1) Lotion: solution titrée de flavonols à 2,5 grammes par litre tamponnée à pH 7, et renfermant un anti-oxydant: 400 ml
ricinoléate de sodium: 10 ml
hygroplex: 5 ml
eau distillée d'hamamélis qsp: 1000 ml
parfum hypoallergique: qs

2) Crème: solution titrée de flavonols à 2,5 grammes par litre, tamponnée à pH 7 et renfermant un anti-oxydant: 1000 ml
huile d'amande: 20 ml
perhydrosqualène: 3 ml
émulsifiant: 20 g
eau distillée de rose qsp: 250 g
parfum hypoallergique: qs

Des essais cliniques effectués ont montré l'efficacité de la composition conforme à l'invention dans les applications cutanées.

L'action régénérante efficace de cette composition vis-à-vis du collagène du tissu conjonctif de la peau, permet en particulier de traiter efficacement les rides.

L'application de la composition conforme à l'invention permet en particulier de faire disparaitre les rides qui apparaissent entre les deux seins à la suite du port du soutiengorge chez les femmes de plus de 50 ans.

Par ailleurs la composition conforme à l'invention présente une action efficace dans le traitement local des varices et des hémorroïdes.

La composition conforme à l'invention peut également être administrée par injection sous-cutanée. On donne ci-après un exemple de formulation d'ampoule injectable:

solution titrée de flavonols à 2,5 grammes par litre tamponnée à pH 7 et renfermant un anti-oxydant: 10 ml

## Revendications

1. Composition pour régénérer le collagène du tissu conjonctif de la peau, renfermant un extrait aqueux d'écorces de conifères contenant des flavonols et de la vitamine C, caractérisée en ce que l'extrait aqueux est tamponné à un pH voisin de 7 et les flavonols sont sous forme de dimères.

2. Composition conforme à la revendication 1, caractérisée en ce que les flavonols sont extraits d'écorces de pin.

3. Composition conforme à l'une des revendications 1 ou 2, caractérisée en ce qu'elle renferme 1 à 5 grammes par litre de flavonols et 10 à 50 mg par litre de vitamine C.

4. Composition conforme à l'une des revendications 1 à 3, caractérisée en ce qu'elle renferme un tampon à base de borate et un anti-oxydant naturel.

5. Procédé pour préparer une composition conforme à l'une des revendications 1 à 4, caractérisé en ce que:

a) on fait infuser à ébullition, puis macérer des écorces de conifères dans de l'eau additionnée d'alcool,

b) on filtre la suspension obtenue et on additionne un anti-oxydant naturel et un tampon pour maintenir le pH à une valeur voisine de 7,

c) on fait macérer des aiguilles de conifères dans de l'eau additionnée d'un tampon pour maintenir le pH à une valeur voisine de 7 puis on filtre et,

d) on mélange le filtrat obtenu avec celui obtenu à la fin de l'étape b).

6. Procédé conforme à la revendication 5, caractérisé en ce qu'on utilise en tant que tampon du borate.

7. Procédé conforme à l'une des revendications 5 ou 6, caractérisé en ce qu'on utilise en tant qu'antioxydant de l'éthyle gallate.

**Patentansprüche**

1. Zusammensetzung zur Regenerierung des Collagens des Bindegewebes der Haut, enthaltend einen wässrigen Koniferenrindenextrakt, der Flavonole und Vitamin C enthält, dadurch gekennzeichnet, daß der wässrige Extrakt auf einen pH-Wert nahe 7 gepuffert ist und die Flavonole in Form von Dimeren vorliegen.

2. Zusammensetzung nach Anspruch 1, dadurch gekennzeichnet, daß die Flavonole Kiefernrindenextrakte sind.

3. Zusammensetzung nach Anspruch 1 oder 2, daduch gekennzeichnet, daß sie 1 bis 5 g Flavonole pro Liter und 10 bis 50 mg Vitamin C pro Liter enthält.

4. Zusammensetzung nach einem der Ansprüche 1 bis 3, dadurch gekennzeichnet, daß sie einen Puffer auf Boratbasis und ein natürliches Antioxidationsmittel enthält.

5. Verfahren zur Herstellung einer Zusammensetzung nach einem der Ansprüche 1 bis 4, dadurch gekennzeichnet, daß man

a) Koniferenrinden in Wasser, dem Alkohol zugesetzt ist, bei Siedetemperatur ziehen läßt und dann mazeriert,

b) die erhaltene Suspension filtriert und ein natürliches Antioxidationsmittel und einen Puffer beigibt, um den pH-Wert nahe 7 zu halten,

c) Koniferennadeln in Wasser einweicht, dem ein Puffer beigegeben ist, um den pH-Wert nahe 7 zu halten, und dann filtriert und

d) das erhaltene Filtrat mit dem am Ende des Schrittes b) erhaltenen mischt.

6. Verfahren nach Anspruch 5, daduch gekennzeichnet, daß man als Puffer Borat einsetzt.

7. Verfahren nach Anspruch 5 oder 6, dadurch gekennzeichnet, daß man als Antioxidationsmittel Äthylgallat einsetzt.

**Claims**

1. A composition for regenerating the collagen of skin connective tissue, containing an aqueous extract of conifer bark containing flavonols and vitamin C, characterised in that the aqueous extract is buffered to a pH of about 7 and the flavonols are in the form of dimers.

2. A composition according to claim 1, characterised in that the flavonols are pine bark extracts.

3. A composition according to claim 1 or 2, characterised in that it contains 1 to 5 grams per litre of flavonols and 10 to 50 mg per litre of vitamin C.

4. A composition according to any one of claims 1 to 3, characterised in that it contains a borate-based buffer and a natural anti-oxidant.

5. A method of preparing a composition according to one of claims 1 to 4, characterised in that:

a) Conifer bark is infused with boiling, and then macerated in water to which alcohol has been added,

b) the resulting suspension is filtered and a natural anti-oxidant and a buffer are added to keep the pH at a value of about 7,

c) conifer needles are macerated in water to which a buffer has been added to keep the pH at a value of about 7, and then filtration is carried out, and

d) the resulting filtrate is mixed with that obtained at the end of step b).

6. A method according to claim 5, characterised in that borate is used as buffer.

7. A method acording to claim 5 or 6, characcterised in that ethyl gallate is used as anti-oxidant.